# EUROPEAN PATENT SPECIFICATION

(11) **EP 0 714 268 B1**
(45) Date of publication and mention of the grant of the patent: **26.02.2003**
(21) Application number: 94925942.8
(22) Date of filing: 15.08.1994
(51) Int. Cl.: A61B 17/22, A61B 17/32, A61B 17/28

(54) **MEDICAL DEVICE WITH IMPROVED ACTUATING HANDLE**
MEDIZINISCHE VORRICHTUNG MIT EINEM VERBESSERTEN HANDBETÄTIGUNGSELEMENT
DISPOSITIF MEDICAL COMPORTANT UNE POIGNEE D'ACTIONNEMENT AMELIOREE

(30) Priority: 19.08.1993 US 109609; 19.10.1993 US 138863
(43) Date of publication of application: 05.06.1996
(73) Proprietor: Boston Scientific Corporation, Natick, Massachusetts 01760 (US)
(72) Inventor: KLINE, Craig, R., Spencer, IN 47460 (US)
(74) Representative: Brunner, Michael John
(86) International application number: PCT/US94/09346
(87) International publication number: WO 95/005129

(56) References cited:
- EP-A- 0 446 020
- US-A- 2 610 631
- US-A- 5 163 942
- US-A- 5 201 740
- US-A- 5 300 078

## Description

This invention relates to polypectomy snares and other actuated medical devices.

There is a need for improved actuation for medical devices. One important example is the surgical snare handle. Snares are used to surgically remove polyps (pre-cancerous tissue growth in the colon and rectum), to remove esophageal carcinoma, and to perform nephrostomy. A snare device is inserted through a working channel of an endoscope fitted with a fiber optic camera, the output of which is attached to a video monitor.

In the case of a polyp, a doctor manipulates the endoscope in the tract of the patient until a polyp is located. The end of the snare in the form of a wire loop is extended and positioned around the polyp, and at the doctor's request, an assistant, typically a nurse, retracts the snare to cause the loop to retract and resect the polyp. An electrical current, such as r-f, is passed through the loop to cauterize the polyp and prevent bleeding.

The same technique is employed for other physical features to be excised or cut by a snare.

The snare is controlled by a snare handle. It is desirable that a snare handle have great fidelity so that whatever resistance is experienced by the snare is felt through the handle by the operator. If the operator cannot feel what is happening at the snare end, the polyp or other tissue might be severed before the cautery current has been applied, causing bleeding and other harmful consequences.

It is desirable that the snare be capable of extending fully and of retracting into the sheath a certain distance as a safety margin, for instance 19mm (0.75 inches). It is also desirable that the handle be constructed so that nurses with small hands can operate it.

Handles having good tactile feeling have long been available. These handles typically have one-to-one activation ratio in which the actuating element of the handle moves the same amount as the snare. Because the distance the snare must move is fairly large, these handles have been difficult to operate with one hand, especially for people with small hands. Attempts have been made to provide a handle which moves only a fraction of the distance of the snare travel. The presence of intervening parts, which may be subject to deformation under load, between the snare loop and the actuating member, however, has rendered the tactile sensitivity of these handles sub-optimal. Furthermore, these handles have also been complex, expensive to manufacture and have other disadvantages.

EP-A-0 446 020 discloses a medical device comprising a body, an actuating member mounted to said body in a manner permitting movement therebetween, a working element remote from said body, and a cable extending between said actuating member and said working element and capable of transmitting bi-directional actuating force between said actuating member and said working element, said actuating member including a pulley surface bodily movable therewith, said cable being trained about said pulley surface such that movement of said actuating member in either direction a given distance, relative to said body, causes said working element to move a substantially greater distance than said given distance.

The present invention is characterised over this prior art by at least one cannula telescopically engaged in said actuating member and at least one cannula telescopically engaged in said body, said cable passing through said cannulae and being supported against columnar buckling by said cannulae.

US-A-2 610 631 discloses a ligator for tying deep ligatures incorporating features similar to that of EP-A-0 446 020.

Preferably, the pulley surface is defined by a rigid formation that is rigidly joined to said actuating member, and said cable portion is slidable relative to said pulley surface during movement of said actuating member.

The flexible cable portion of said cable may be substantially confined throughout its length, enabling transmission of axial thrust on said working element.

Preferably, said body includes a rigid proximal portion adjacent to said actuating member and an elongate flexible distal portion through which said cable extends.

The elongated flexible distal portion may comprise a medical catheter.

The cable may be a flexible metal cable.

Preferably, said body terminates in a thumb loop centrally located on said body, and said actuating member defines two finger loops disposed on opposite sides of said thump loop.

The body may further comprise an electrical terminal to which said cable is attached, said electrical terminal being capable of transmitting an electrical cauterizing current.

The working element may be any one of a snare loop, an angled snare loop, a grasper or a basket.

The present invention will now be described with reference to the drawings in which:
FIG. 1 is a top view, partially broken away, of a preferred embodiment of the invention.
FIG. 1A is a transverse cross-sectional view on an enlarged scale taken on line 1a - 1a of FIG. 1.
FIG. 2 is a longitudinal, cross-sectional view, partially broken away, of the preferred embodiment of FIG. 1 taken on line 2-2 of FIG. 1.
FIG. 2A is a cross-sectional view on an enlarged scale of the portion of the device in FIG. 2 encircled-by the chained lines.
FIG. 3 is a view similar to FIG. 2 showing the positions of the handle of the device during actuation and depicting the snare in solid lines in its extended position.
FIG. 4 is a perspective view of the embodiment of Figs. 1-3 illustrating the two positions of the handle as indicated in FIG. 3, with the handle in solid lines in the retracted position.
FIG. 5 is a view similar to FIG. 4 illustrating the position achieved by the snare after actuation in which the snare has retracted into the tube of the device.
FIGs. 6 and 7 are views similar to FIG. 2 showing alternate embodiments of the invention.
FIG. 7a is a longitudinal, cross-sectional view, of the embodiment of FIG. 7, taken on line 7a - 7a of Fig. 7.
FIGs. 8 - 18 are perspective views of alternate working elements which may be used in conjunction with the invention.
FIG. 19 is a longitudinal, cross-sectional view, partially broken away, of a working element of an embodiment of the invention as used in a syringe.
FIG. 20 is a perspective view, partially broken away, of an embodiment of the invention as used with a balloon catheter. Fig. 20a is a cross section taken on line 20a - 20a of Fig. 20.

### Description of Preferred Embodiments

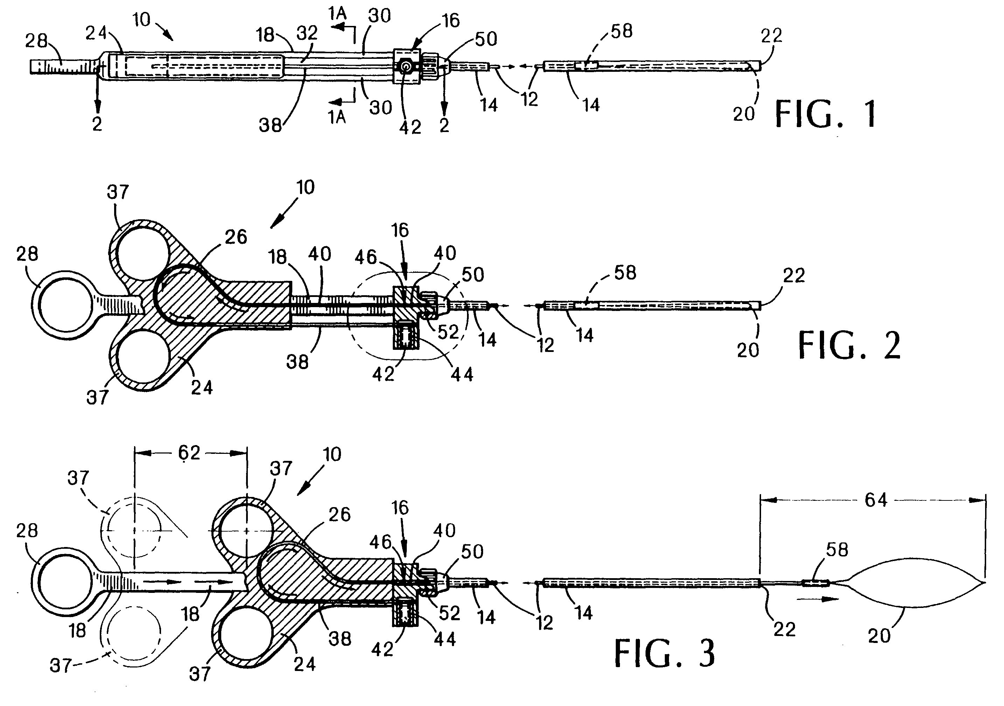

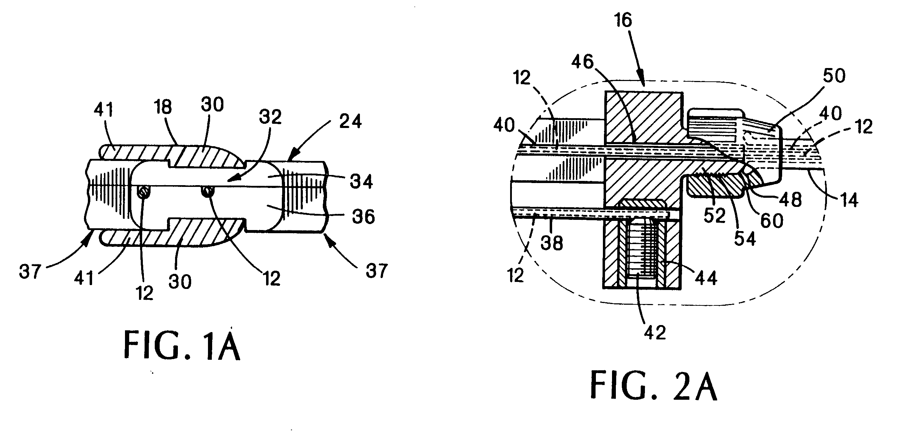

Referring now generally to FIGs. 1-3, snare handle 10 advances and withdraws cable 12 within sheath 14. The proximal end of cable 12 is fixed to nose 16 of handle body 18. At the distal end of cable 12 is snare loop 20, which can be used to excise a polyp (not shown) by advancing snare loop 20 beyond tip 22 of sheath 14, looping snare loop 20 around the polyp, and then retracting snare loop 20 into sheath 14 such that snare loop 20 is entirely retracted through sheath tip 22.

Handle body 18, preferably, is a single injection molded component composed of nylon or other resilient material having legs 30 which define channel 32. A thumb ring 28 is located at one end of handle body 18 to enable an operator to control it, and, if desired, thumb ring 28 may be rotatable.

Referring more specifically to FIG. 1a, actuating member 24 is slidably disposed on handle body 18 between legs 30 in channel 32. Actuating member 24 preferably is made of nylon, DELRYN, or any suitable polymeric injection molded material. Actuating member 24 may be manufactured as a clam shell with top half 34 and bottom half 36 separately injection molded and later joined by melting, snap fitting, through the application of an adhesive such as cyanoacrylate, or clamped together by legs 30 of handle body 18. As shown in FIGS. 1-3, actuating member 24 preferably has two finger loops disposed on opposite sides of handle body 18 for engagement by fingers of an operator.

Pulley surface 26 is disposed on actuating member 24 and may be rotary or stationary. In a preferred embodiment, pulley surface 26 defines a rigid, semicircular track integral with actuating member 24 and has a cross section only slightly larger than cable 12 to prevent cable 12 from buckling under compressive force.

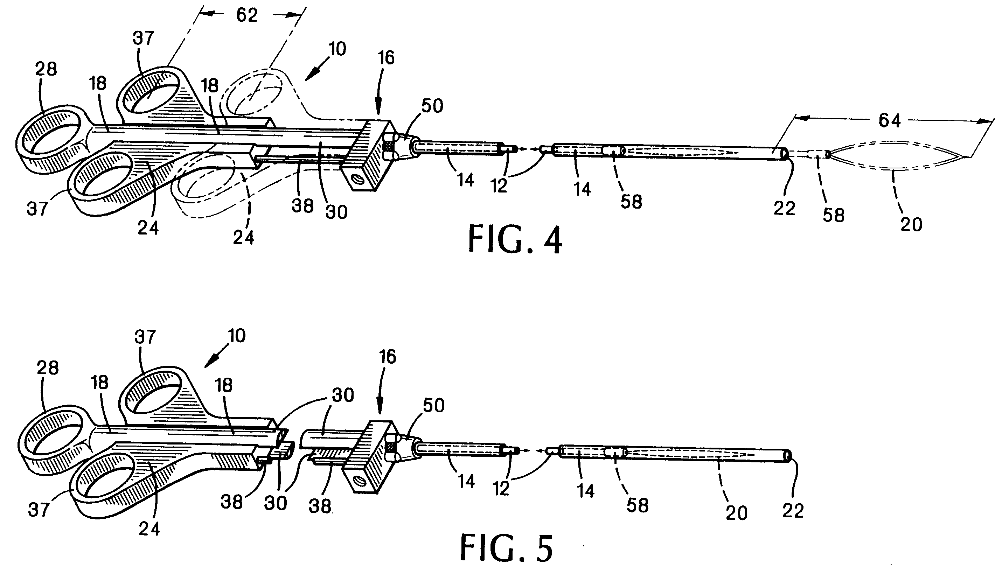

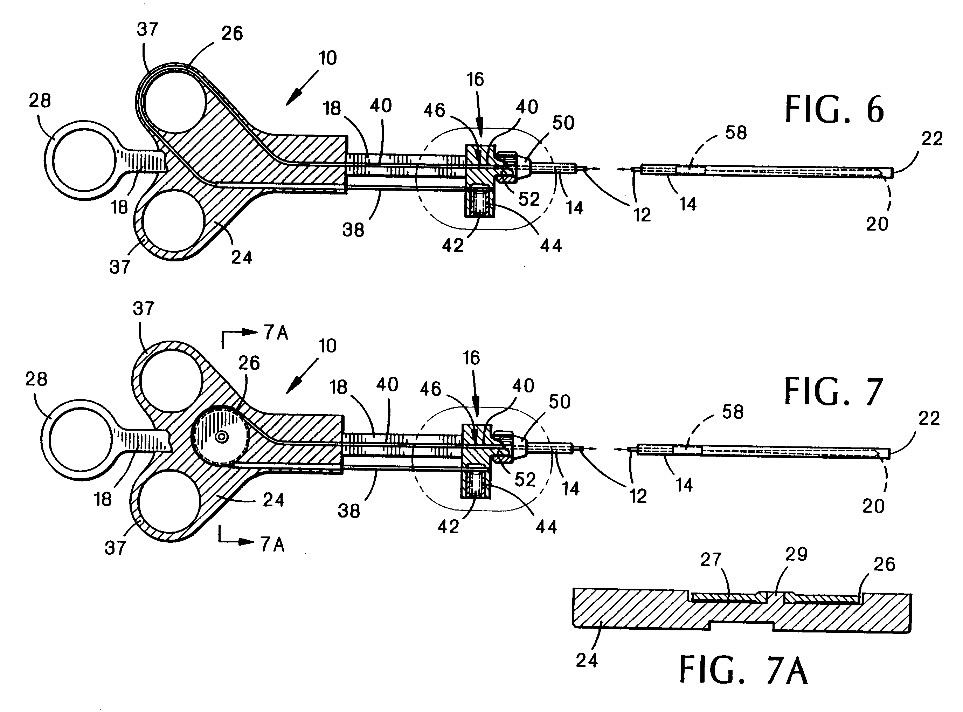

Pulley surface 26 may be located as shown in FIGs. 2 and 3 or may be located at another position on actuating member 24, such as the embodiment shown in FIG. 6, where pulley surface 26 corresponds with finger hole 37.

Pulley surface 26 may also be rotatable as shown in FIGs. 7 and 7a. In this embodiment, pulley 27 is rotatably mounted on axle 29. Pulley surface 26 may be flat or indented to accommodate cable 12.

A biocompatible silicon type lubricant may be used to reduce friction on pulley surface 26 in embodiments of the invention employing both fixed and rotatable pulley surfaces.

The pulley advantage of pulley surface 26, which is in direct contact with cable 12, allows the operator to achieve the desired two to one activation ratio, while the absence of intervening parts provides the tactile feeling previously only available in handles with a one to one activation ratio.

Referring now to FIG. 2A, cable 12 passes through nose 16 of handle body 18 around pulley surface 26 on actuating member 24, and back to nose 16 where the end of cable 12 is fixed to nose 16. Cable 12 is confined throughout its entire pathway within medical device 10 to prevent columnar buckling. Preferably, cannulae 38 and 40 are used to confine cable 12 between nose 16 and actuating member 24. Cable 12 is connected, by soldering or any other method, to one end of cannula 38. This end is attached to body 18 by set screw 42 disposed in can 44 on nose 16. The other end of cannula 38 is telescopically disposed within actuating member 24. Similarly, cannula 40 has one end attached to actuating member 24 and the other end telescopically disposed within aperture 46 in nose 16.

Referring now to FIG. 3, actuation of the snare handle causes cable 12 to move about pulley surface 26, through cannula 40 and out nose 16. Due to the pulley advantage of pulley surface 26, movement of actuating member 24 a given distance 62 results in translation of cable 12 twice the given distance 64. Similarly, when actuating member 24 is retracted, cable 12 retracts twice as far as the movement of actuating member 24 due to the pulley advantage of pulley surface 26. This two-fold increase in the cable stroke relative to movement of actuating member 24 permits a nurse with small hands to operate the snare handle with one hand.

Cable 12 is preferably a stainless steel braided cable that is quite stiff and not prone to buckle under compression. Cable 12 could, however, be any other electrically conductive, such as single strand wire, which is capable of withstanding both tensile and compressive forces and may have rigid portions. The cable currently contemplated to be used be the invention has a diameter from 0.25mm - 1.52mm (10 to 60 thousandths of an inch) and more preferably from 0.76mm - 1.02mm (30 to 40 thousandths), although other cable diameters may be acceptable.

Cannulae 38 and 40 are preferably stainless steel tubes which confine cable 12 and allow about 0.13mm (five thousandths of an inch) clearance between the inside wall of the tube and the cable. The cannula wall is preferably about 0.05mm (two thousandths of an inch) thick. The exterior of cannula 38 and 40 may be coated with an insulator such as nylon to prevent a person operating the medical device from being shocked. Alternatively, as shown in FIG. 1A, guard 41 (a part of body 18 which extends outward from legs 30 to cover cannula 38) may be included for this same purpose.

Referring now to FIG. 2A, set screw 42 attaches cannula 38 to body 18 such that an electrical connection forms. If a connector is attached to the set screw, an electrical current, such as r-f, may be applied to cable 12, thereby allowing for electrical cauterization of a polyp by snare loop 20.

Sheath 14 has flared end 48 which is attached by compression cap 50 to side extension 52 of nose 16. Side extension 52 carries a plurality of threads 54 on an outer surface and has beveled front 60 which mates with flared end 48. Compression cap 50 is threaded onto side extension 52 and traps flared end 48 against beveled front 60.

The inside of sheath 14 aligns with aperture 46 in nose 16 and is large enough that cannula 40 may be telescopically disposed in it, but must also be small enough to prevent cable 12 from buckling upon application of compressive force. Sheath 14 is preferably made of teflon or another lubricous flexible material which does not conduct electricity. Tip 22 of sheath 14 may be cut on any desired angle.

Referring now to FIG. 3, snare loop 20 is preferably attached to the distal end of cable 12 by crimp connector 58, however, another means of connection, such as soldering or brazing, may be used. It is desirable that this connection be capable of conducting electricity from cable 12 to snare loop 20.

Snare loop 20 is preferably stainless steel braided wire of a lesser diameter than cable 12, usually from 0.51mm - 1.52mm (20 to 40 thousandths of an inch) in diameter. Snare loop 20 should be flexible to withdraw into the end of sheath 14, and should be resilient and retain its shape when extended.

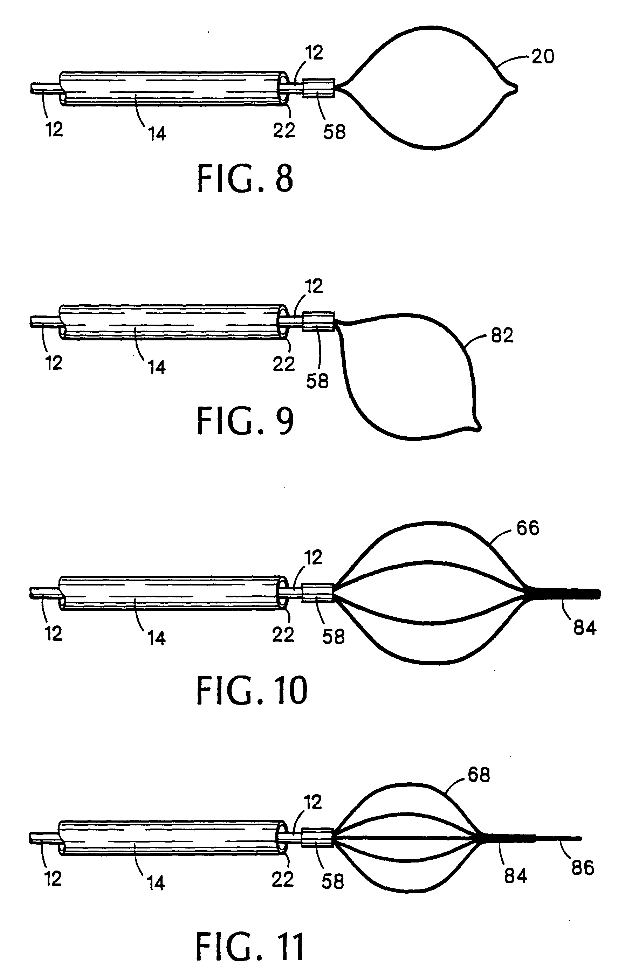

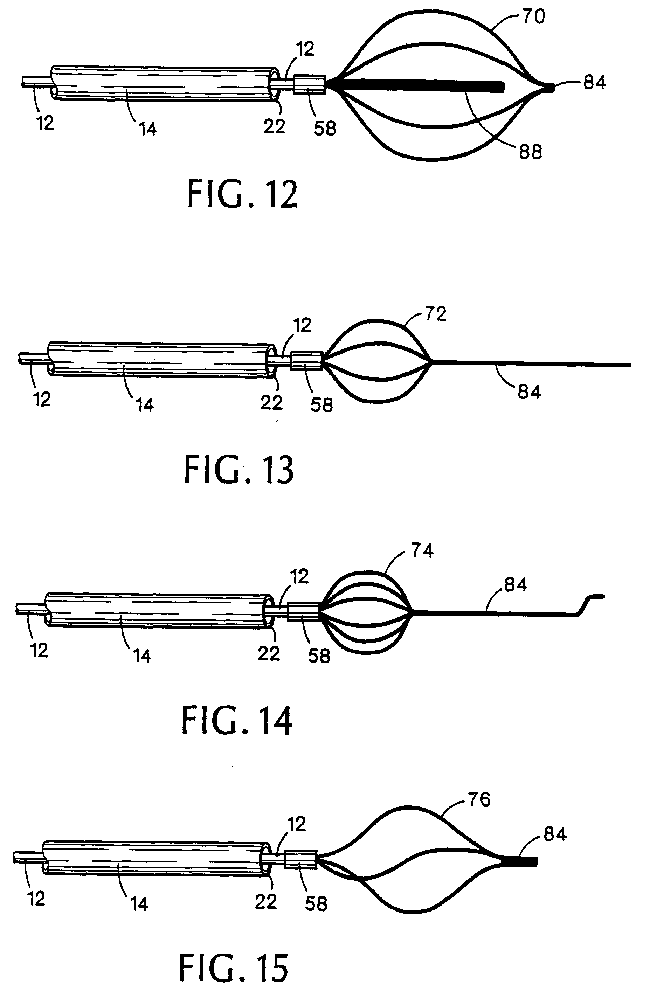

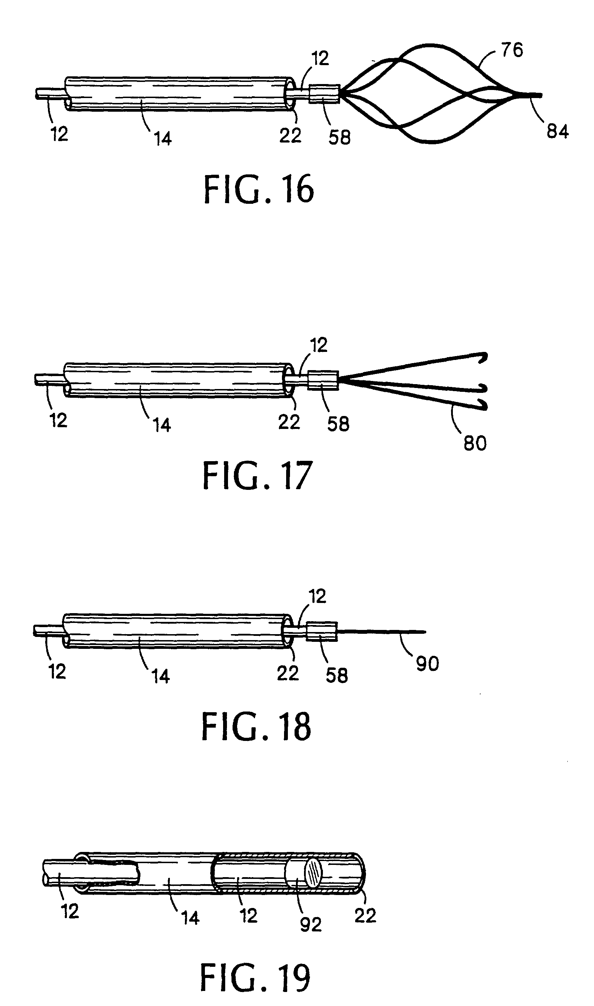

As shown in FIGs 8-18, other types of working elements such as baskets 66-79, grasping forceps 80, right angle snare loop 82, or point type cauterization device 90 may be substituted for snare loop 20. Baskets 66-78 may have tips 84 of any desired length or shape, and the number of wires may vary, although usually four or six wires are used. As shown in FIG. 11, basket, 68 may be designed to slide upon wire 86, or, as shown in FIG. 12, basket 68 may be designed to accommodate pulsed-dye laser fibre-optic probe 88. Grasping forceps 80 may have three prongs as shown in FIG. 17 or any other desired number of prongs. Point type cauterization device 90 may be any type of needle, wire or electrode.

The snare handle described above may also be useful in other areas of medicine, such as laparoscopic surgery, or as a syringe handle. It occasionally is necessary to pickup or deliver very small amounts of drugs such as Tpa. As shown in FIG. 19, when the medical device of the invention is used as a syringe, sheath 12 is reduced in size sp that the inside diameter of sheath 14 is substantially identical to the diameter of cable 12. Preferably, the clearance between sheath 14 and cable 12 would be 0.03mm - 0.05mm (one to two thousandths of an inch). The working element in this embodiment of the invention is a device such as plug 92, which is disposed on the end of cable 12 such that a seal is formed between plug 92 and the inside of sheath 14. When the cable is withdrawn, this causes a suction which draws fluid into the end of sheath 12. Preferably, the length of cable 12 is adjusted sp that the plug 92 remains within the sheath 14 regardless of the position of the actuating member 24 on body 18. The length of cable 12 must also be adjusted so that plug 92 will extend completely to tip 22 of sheath 14. Plug 92 may be any type of material, such as rubber, which will cause a seal to form.

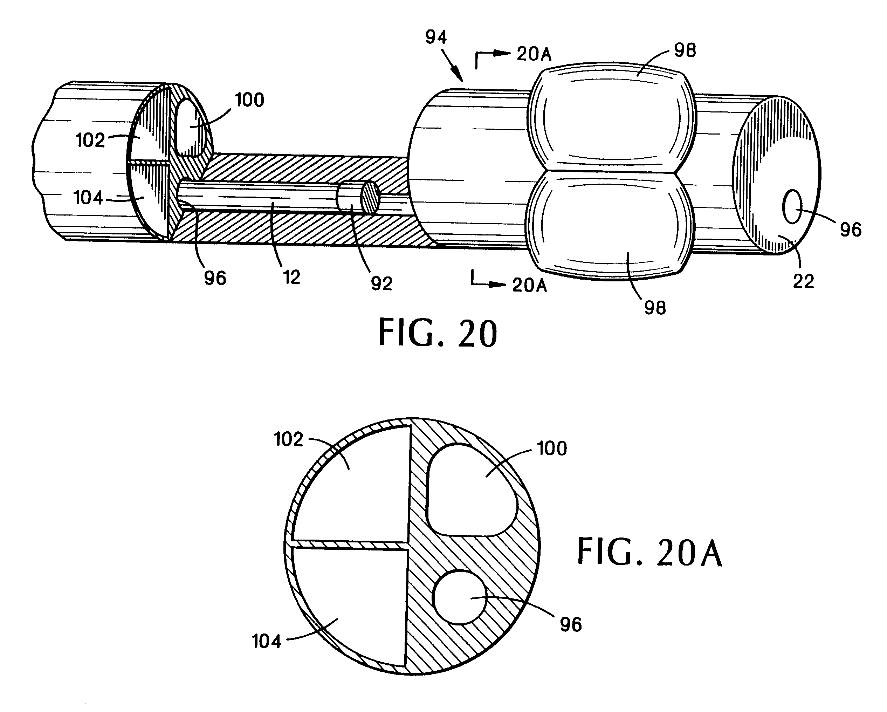

As shown in FIGs 20. And 20a, the medical device of the invention could be used in conjunction with a single balloon or multi-balloon catheter 94 by inserting cable 12 and associated working element, preferably plug 92, through lumen 96. Balloons 98 are inflated by the introduction of fluid through lumens 100, 102 and 104 which may be of any desired shape. Lumens 100, 102 and 104 preferably run the length of balloon catheter 94, but do not intersect catheter end 22.

## Claims

1. A medical device comprising a body (18), an actuating member (24) mounted to said body in a manner permitting movement therebetween, a working element (20) remote from said body (18), and a cable (12) extending between said actuating member (24) and said working element (20) and capable of transmitting bi-directional actuating force between said actuating member and said working element (20), said actuating member including a pulley surface (26) bodily movable therewith, said cable (12) being trained about said pulley surface such that movement of said actuating member (24) in either direction a given distance (62), relative to said body (18), causes said working element (20) to move a substantially greater distance (64) than said given distance, **characterised by**
at least one cannula (40) telescopically engaged in said actuating member (24) and at least one cannula (38) telescopically engaged in said body (18), said cable (12)passing through said cannulae and being supported against columnar buckling by said cannulae (38, 40).

2. The medical device of claim 1, wherein said pulley surface (26) is defined by a rigid formation that is rigidly joined to said actuating member (24), and said cable (12) portion is slidable relative to said pulley surface (26) during movement of said actuating member (24).

3. The medical device of claim 1 or claim 2, wherein the flexible cable (12) portion of said cable (12) is substantially confined throughout its length, enabling transmission of axial thrust on said working element (20, 66-78, 80, 82, 90).

4. The medical device of any of claims 1 to 3, wherein said body (18) includes a rigid proximal portion adjacent to said actuating member (24) and an elongate flexible distal portion through which said cable (12) extends.

5. The medical device of claim 4, wherein said elongate flexible distal portion comprises a medical catheter (94).

6. The medical device of claim 1, wherein said cable is a flexible metal cable.

7. The medical device of any of claims 1 to 6, wherein said body (18) terminates in a relatively stationary thumb loop (28) centrally located on said body (18), and said actuating member (24) defines two finger loops (37) disposed on opposite sides of said thump loop (28).

8. The medical device of any of claims 1 to 7, wherein said body (18) further comprises an electrical terminal (42) to which said cable (12) is attached, said electrical terminal (42) being capable of transmitting an electrical cauterizing current.

9. The medical device of any of claims 1 to 8, wherein said working element is a snare loop (20).

10. The medical device of claim 9, wherein said snare loop is an angled snare loop (82).

11. The medical device of any of claims 1 to 8, wherein said working element is a grasper (80).

12. The medical device of any of claims 1 to 8, wherein said working element is a basket (66-78).

## Patentansprüche

1. Medizinische Vorrichtung, aufweisend einen Körper (18), ein Betätigungselement (24), welches an den Körper in einer Art und Weise montiert ist, dass zwischen diesen eine Bewegung gestattet wird, ein Arbeitselement (20) entfernt von dem Körper (18), und ein Kabel (12), welches sich zwischen dem Betätigungselement (24) und dem Arbeitselement (20) erstreckt und geeignet ist, eine bi-direktionale Betätigungskraft zwischen dem Betätigungselement und dem Arbeitselement (20) zu übertragen, wobei das Betätigungselement eine Riemenoberfläche (26) umfasst, welche körperlich damit bewegbar ist, wobei das Kabel (12) um diese Riemenoberfläche derart geführt ist, dass die Bewegung des Betätigungselements (24) in jeder Richtung einer gegebenen Distanz (62), relativ zu dem Körper (18), bewirkt, dass das Arbeitselement (20) sich im Wesentlichen um eine größere Distanz (64) als die gegebene Distanz bewegt, **gekennzeichnet durch** zumindest eine Kanüle (40), die teleskopartig mit dem Betätigungselement (24) in Eingriff steht, und zumindest eine Kanüle (38), die teleskopartig mit dem Körper (18) in Eingriff steht, wobei das Kabel (12) **durch** diese Kanülen wandert und gegen säulenartiges Knicken **durch** diese Kanülen (38, 40) gestützt wird.

2. Medizinische Vorrichtung nach Anspruch 1, wobei die Riemenoberfläche (26) durch eine steife Formation definiert ist, welche fest mit dem Betätigungselement (24) verbunden ist, und wobei ein Abschnitt des Kabels (12) gegenüber der Riemenoberfläche (26) während der Bewegung des Betätigungselements (24) gleitfähig ist.

3. Medizinische Vorrichtung nach Anspruch 1 oder 2, wobei der flexible Kabelabschnitt des Kabels (12) im Wesentlichen durch seine Länge begrenzt ist, was die Übertragung eines axialen Schubs auf das Arbeitselement (20, 66-78, 80, 82, 90) ermöglicht.

4. Medizinische Vorrichtung nach einem der Ansprüche 1 bis 3, wobei der Körper (18) einen festen proximalen Abschnitt in der Nähe des Betätigungselements (24) und einen länglichen flexiblen distalen Abschnitt umfasst, durch welchen sich das Kabel (12) erstreckt.

5. Medizinische Vorrichtung nach Anspruch 4, wobei der längliche flexible distale Abschnitt einen medizinischen Katheter (94) aufweist.

6. Medizinische Vorrichtung nach Anspruch 1, wobei das Kabel ein flexibles Metallkabel ist.

7. Medizinische Vorrichtung nach einem der Ansprüche 1 bis 6, wobei der Körper (18) in einer relativ stationären Daumenschlaufe (28) endet, welche mittig auf dem Körper (18) lokalisiert ist, und wobei das Betätigungselement (24) zwei Fingerschlaufen (37) definiert, die an gegenüberliegenden Seiten der Daumenschlaufe (28) vorgesehen sind.

8. Medizinische Vorrichtung nach einem der Ansprüche 1 bis 7, wobei der Körper (18) des Weiteren einen elektrischen Anschluss (42) aufweist, an welchem das Kabel (12) angebracht ist, wobei der elektrische Anschluss (42) geeignet ist, um einen elektrischen Strom zum Veröden zu übertragen.

9. Medizinische Vorrichtung nach einem der Ansprüche 1 bis 8, wobei das Arbeitselement eine Schlingenschlaufe (20) ist.

10. Medizinische Vorrichtung nach Anspruch 9, wobei die Schlingenschlaufe eine angewinkelte Schlingenschlaufe (82) ist.

11. Medizinische Vorrichtung nach einem der Ansprüche 1 bis 8, wobei das Arbeitselement ein Greifer (80) ist.

12. Medizinische Vorrichtung nach einem der Ansprüche 1 bis 8, wobei das Arbeitselement ein Korb (66-78) ist.

## Revendications

1. Dispositif médical comprenant un corps (18), un élément d'actionnement (24) monté sur ledit corps d'une manière autorisant le mouvement intermédiaire, un élément de travail (20) éloigné dudit corps (18), et un câble (12) s'étendant entre ledit élément d'actionnement (24) et ledit élément de travail (20) et qui est apte à transmettre une force d'actionnement bidirectionnelle entre ledit élément d'actionnement et ledit élément de travail (20), ledit élément d'actionnement comprenant une surface de poulie (26) déplaçable solidairement avec celui-ci, ledit câble (12) étant engrené sur ladite surface de poulie (24) de sorte que le mouvement dudit élément d'actionnement (24) dans une direction ou une autre sur une distance donnée (62), relative audit corps (18), a pour effet que ledit élément de travail (20) se déplace sur une, distance sensiblement plus grande (64) que ladite distance donnée, **caractérisé par**
au moins une canule (40) engagée télescopiquement dans ledit élément d'actionnement (24) et au moins une canule (38) engagée télescopiquement dans ledit corps (18), ledit câble (12) traversant ladite canule et étant supporté pour éviter le flambage colonnaire par lesdites canules (38,40).

2. Dispositif médical selon la revendication 1, dans lequel ladite surface de poulie (26) est définie par une formation rigide qui est reliée rigidement audit élément d'actionnement (24), et ladite portion de câble (12) peut coulisser par rapport à ladite surface de poulie (26) au cours du mouvement dudit élément d'actionnement (24).

3. Dispositif médical selon la revendication 1 ou la revendication 2, dans lequel la portion de câble souple (12) dudit câble (12) est sensiblement confinée sur toute sa longueur, permettant la transmission de la poussée axiale sur ledit élément de travail (20,66-78,80,82,90).

4. Dispositif médical selon l'une quelconque des revendications 1 à 3, dans lequel ledit corps (18) comprend une portion proximale rigide contiguë audit élément d'actionnement (24) et une portion distale souple oblongue par laquelle s'étend ledit câble (12).

5. Dispositif médical selon la revendication 4, dans lequel ladite portion distale souple oblongue comprend un cathéter médical (94).

6. Dispositif médical selon la revendication 1, dans lequel ledit câble est un câble métallique souple.

7. Dispositif médical selon l'une quelconque des revendications 1 à 6, dans lequel ledit corps (18) se termine par une boucle relativement fixe pour l'engagement du pouce (28) située centralement de façon relativement fixe sur ledit corps (18), et ledit élément d'actionnement (24) définit deux boucles pour l'engagement des doigts (37) disposées sur les côtés opposés de ladite boucle pour l'engagement du pouce (28).

8. Dispositif médical selon l'une quelconque des revendications 1 à 7, dans lequel ledit corps (18) comprend de plus une borne électrique (42) sur laquelle est fixé ledit câble (12), ladite borne électrique (42) étant apte à transmettre un courant de cautérisation électrique.

9. Dispositif médical selon l'une quelconque des revendications 1 à 8, dans lequel ledit élément de travail est une boucle à noeud coulant (20).

10. Dispositif médical selon la revendication 9, dans lequel ladite boucle à noeud coulant est une boucle à noeud coulant angulaire (82).

11. Dispositif médical selon l'une quelconque des revendications 1 à 8, dans lequel ledit élément de travail est un préhenseur (80).

12. Dispositif médical selon l'une quelconque des revendications 1 à 8, dans lequel ledit élément de travail est un panier (66-78).
